# EUROPEAN PATENT SPECIFICATION

(11) **EP 2 324 797 B1**
(45) Date of publication and mention of the grant of the patent: **08.05.2019**
(21) Application number: 10191069.3
(22) Date of filing: 12.11.2010
(51) Int. Cl.: A61F 2/16

(54) **Injector tip for an injector device as well as injector device for introducing an intraocular lens into an eye**
Einspritzspitze für eine Einspritzvorrichtung und Einspritzvorrichtung zum Einführen einer Intraokularlinse in ein Auge
Pointe d'injecteur pour dispositif injecteur ainsi que dispositif injecteur pour introduire une lentille intra-oculaire dans un oeil

(30) Priority: 20.11.2009 GB 0921104
(43) Date of publication of application: 25.05.2011
(73) Proprietor: Carl Zeiss Meditec SAS, 17053 La Rochelle Cedex 9 (FR)
(72) Inventor: Rathert, Brian, 10115 Berlin (DE); Lesage, Cédric, 17220 Sainte-Soulle (FR); Raquin, Vincent, 17000 La Rochelle (FR); Gatto, Alexandre, 07751 Jena Münchenroda (DE); Pankin, Dmitry, 13355 Berlin (DE)
(74) Representative: Hofstetter, Schurack & Partner

(56) References cited:
- EP-A1- 1 338 254
- WO-A1-01/39701
- US-A- 5 928 245

## Description

### Technical field

The invention relates to an injector tip for an injector device for introducing an intraocular lens into an eye, which has a continuous guide channel for the intraocular lens. Furthermore, the invention relates to an injector device with such an injector tip.

### Prior art

Intraocular lenses are inserted into an eye as implants and replace the natural lens. For this, injector devices are provided, which have a plunger guided in an injector tube. At the front end of the injector tube, a receiving space for the intraocular lens is formed, wherein this receiving space can be formed in a separate cassette, which can be introduced into a frame of the injector tube. It can also be provided that the receiving space is integrally formed in the injector tube. Moreover, following the receiving space towards the front, an injector tip is formed, which has a guide channel, in which the intraocular lens is advanced through after advancing out of the receiving space and exits at the front in a folded state and is inserted into the eye. The front side of the tip is directly introduced into the eye.

In known intraocular lenses, with conventional known injector tips and injector devices, there arises the problem that they either curl up in uncoordinated manner with regard to their folding such that in particular with asymmetrical lenses having an optic part with differently curved surfaces the folding optionally is effected in an undesired incorrect direction.

Furthermore, from EP 0 722 292 B1, a device for inserting a flexible intraocular lens into an eye is known. There, an injector tip is shown, which only has a simple oval cross-section, which has an outwardly curved top curve, lateral curves joining thereto and a bottom curve. In the configuration of the injector device known there, in front of this tip having a three-part segmented construction in the form of a cannula, a receiving space is formed, in which the intraocular lens is disposed in its rest position before advancing-out. Therein, the arrangement is such that it is horizontally oriented with its center plane. This receiving space is confined towards the top by a cover, wherein already this receiving space is very short viewed in longitudinal direction and has a varied cross-sectional shaping. This shaping is relatively small in its clear height such that the intraocular lens is disposed therein nearly exactly fitting and is folded. Thus, the intraocular lens already folded enters the injector tip, wherein problems arise in transferring from the receiving space into the injector tip, which considerably tapers, in this respect. The folded lens can undesirably deform upon entering the injector tip, and the achieved folded position can again come undone or a ball or plug formation of the lens occurs. The tip circumferentially surrounds the cover and further components constituting the receiving space with its rear proximal section on the outside. Such an intense folding of the lens in the receiving space and thus over a relatively short path along the longitudinal axis involves considerable problems as it is explained in EP 1 233 730 B1, which is based on this above-mentioned prior art in its invention.

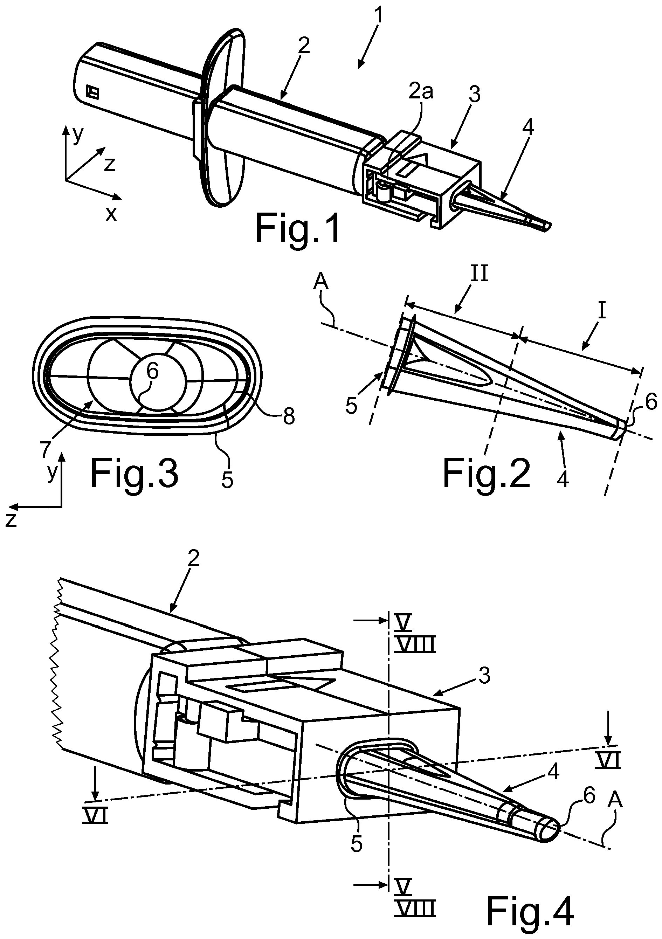

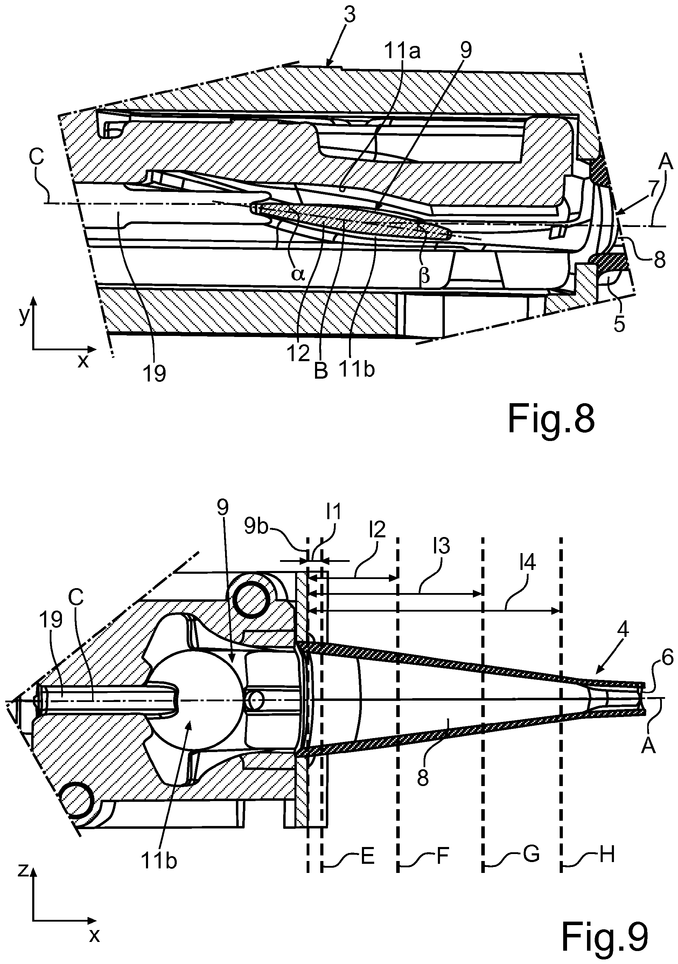

In EP 1 233 730 B1, an implantation device for intraocular lenses is shown. There, cross-sectional illustrations are shown in fig. 2a to 2e, which represent a narrow configuration viewed in vertical direction immediately following the receiving space such that here too, the lens is inserted virtually fitting exactly with its thickness. Moreover, the exterior edges are formed with very small radiuses such that here too, the edge regions of the lens are encompassed virtually fitting exactly. By such cross-sectional shapings, in folding, the lens is subjected to extremely high mechanical stresses in particular on its exterior regions such that in particular at these locations damages to the lens in the folding operation can occur in this respect. In particular also at the end of the guide channel according to the configurations in fig. 2d and 2e therein, exterior edge regions of the lens are virtually flatly pressed such that the lateral edges of the lens are possibly located above the optic part of the lens, but the lateral edges face each other. In particular at the folding locations, therefore, at the end of folding, particularly high mechanical stresses are exerted to the lens along the cross-sectional configurations, which is considerably disadvantageous. The same applies to the implementations in fig. 9g to 9j of EP 1 233 730 B1l.

US 5,928,245 A discloses an injector tip, with a guide channel in which an intraocular lens is folded. In a cross section the wall of the channel has a protrusion on the top wall section.

### Presentation of the invention

It is the object of the present invention to provide an injector tip for an injector device as well as an injector device with such an injector tip , by which a secure and reliable folding of a lens in a desired direction is effected, and therein mechanical elongation and tensile stresses to the lens as low as possible, in particular at the folding locations, occur.

This object is solved by injector tips having the features according to claim 1.

An injector tip according to the invention according to a first aspect of the invention is formed for an injector device for introducing an intraocular lens into an eye. The injector tip has a continuous guide channel for the intraocular lens. In cross-section, the guide channel is formed at least in sections along a rear second half with a contour development, which has at least two outwardly curved top curves or side roof arcs joining to each two outwardly curved lateral curves or side arcs. Moreover, the contour development includes an outwardly curved bottom curve or bottom arc connected to the lateral curves. A lateral curve and an adjoining top curve are formed such that at least in the second half viewed along the longitudinal axis of the guide channel, a horizontal connecting straight line between a minimum of the top contour constituting a first end of the top curve and a second end of the top curve, and a vertical connecting straight line between two endpoints of a lateral curve are formed such that a length ratio between the vertical connecting straight line and the horizontal connecting straight line is between 0.7 and 0.95 in the second half of the guide channel. Especially said connecting straight lines intersecting on the contour development. Thus, the guide channel is formed with relatively large top curves and relatively large lateral curves in its cross-sectional profile at least in the rear second half of the guide channel. By such shaping in particular on the side and in the top region of the guide channel in this half of the injector tip facing a receiving space for an intraocular lens, a relatively large volume is provided. Thereby, it can be achieved on the one hand that the intraocular lens is guided in the guide channel not virtually exactly fitting, and a certain clearance and movement space for the lens is present in this respect. However, on the other hand, the lateral curves are sized relatively large and outwardly curved, wherein the top curves are also correspondingly formed in this respect. By these indicated length ratios, a top curve can result in a particularly uniform and large curve configuration with a size as similar and adapted to each other as possible on an adjoining lateral curve such that the folding of the lens is effected with a mechanical stress as low as possible. In particular at the folding locations on the exterior region of the lens, thus, the mechanic effect due to elongation and bending is considerably reduced with respect to the prior art such that folding damages of the lens are avoided.

Preferably, the guide channel has a shaping confining the internal space of the channel, by which an intraocular lens is folded at least at a location in a front first half viewed along the longitudinal axis of the guide channel, such that opposing lateral edges of the intraocular lens face a top side of an optic part of the intraocular lens. By such a shaping of the guide channel, thus, it is achieved that the lens is folded in a desired correct direction on the one hand, and the lateral edges are virtually upwards folded in this respect. In that the shaping explicitly is also such that the exterior lateral edges of the intraocular lens are then also disposed pointing to the top, the lens can be formed especially with uniform curve geometry in the folding region such that here too, a relatively uniform and reduced mechanical stress occurs along the folding regions, and particularly high mechanical stresses will not occur at some specific locations, as it is the case in the prior art. Not least, by such a shaping of the guide channel with folding position of the intraocular lens in this respect, a particularly suitable position can be achieved to the effect that the folded state is securely retained and a corresponding introduction of the lens into the eye can be achieved securely and precisely with this desired shaping.

A further injector tip according to the invention according to a second aspect is also provided for an injector device for introducing an intraocular lens into an eye. The injector tip has a continuous guide channel for the intraocular lens. In cross-section, the guide channel is formed at least in sections with a contour development with at least two outwardly curved top curves or roof arcs, two outwardly curved lateral curves or side arcs and an outwardly curved bottom curve or bottom arc. The guide channel has a shaped wall confining the internal space of the channel, by which an intraocular lens is folded at least at a location in a front first half viewed along the longitudinal axis of the guide channel such that opposing lateral edges of the intraocular lens face a top side of an optic part of the intraocular lens. By this injector tip, a configuration is also allowed, in which a particularly reliable and secure desired folding direction of the lens is achieved during the folding operation of the lens in the injector tip, and moreover a particularly suitable configuration with regard to undesired elongations and bendings of the lens in the folding operation is achieved. In particular in the front region of the guide channel, thus, a folding position of the lens in cross-section is achieved, which is associated with an extremely low mechanical stress of the lens, and in particular the bent lateral regions of the optic part of the lens can be retained in the guide channel with mechanical stress as low as possible. Thereby, it is not only ensured that undesired damages to the lens can be avoided in this respect, rather, a folding position at the exit of the guide channel is thereby also achieved, which ensures secure and reliable introduction of the lens into the eye with a particularly suitable folded shape of the lens. In particular, this folded shape achieved due to the shaped wall of the contour development of the guide channel in the first half is achieved with a relatively uniform arcuate configuration of the bent lateral regions of the lens. Thereby, undesirably greatly bent or kinked regions in the folded shape are avoided.

Preferably, in a second aspect, it is provided on an injector tip that the shaping of the guide channel in cross-section has at least in sections along a rear second half a contour development with at least two outwardly curved top curves, to which an outwardly curved lateral curve immediately joins, respectively, which are directly connected by an outwardly curved bottom curve. At least in the second half, a horizontal connecting straight line between a minimum of the top contour constituting a first end of a top curve and a second end of the top curve, and a vertical connecting straight line between two endpoints of a second curve are formed. A length ratio between the vertical and the horizontal connecting straight line is between 0.7 and 0.95 in the second half. Especially said connecting straight lines intersecting on the contour development. The advantages to be achieved in this respect are mentioned according to the configuration of the injector tip according to the invention according to the first aspect.

The advantageous embodiments mentioned afterwards in the following can be provided for both aspects of the mentioned injector tips.

Thus, in particular, the lateral regions of the intraocular lens are formed as relatively uniformly curved curves in that position, in which the lateral edges face the top side or already rest on it. Thus, virtually uniform mechanic elongation and tensile effects are produced over the substantially entire curve length of these regions of the lens bent in such manner, which is not possible with the cross-sectional geometries in the prior art upon foldings.

Thus, it can be provided that the lateral curves of the contour development are formed at least in the second half such that a length of a vertical connecting straight line between the two endpoints of a lateral curve is varied by a maximum of 20 %, in particular a maximum of 10 %, along the longitudinal axis of the injector tip. This in particular means that a lateral curve only very minimally varies with its height extension along the longitudinal axis of the guide channel such that in particular also by this configuration a very steady shaping results, which effects a particularly gentle folding without abrupt or very great mechanical force effects on the lens due to elongation and bending. By this advantageous configuration, there is in particular provided the option, in which the length difference differs by not more than 20 %, in particular not more than 10 %, at two arbitrarily taken locations along the longitudinal axis of the guide channel at least in the second half. By such a configuration, relatively large lateral curves with regard to their height can be provided, which vary only relatively slightly along the longitudinal axis at least in the second half.

Preferably, it is provided that the lateral curves are formed substantially over the entire length of the guide channel such that a length of a vertical connecting straight line between the two endpoints of a lateral curve is varied by a maximum of 20 %, in particular a maximum of 10 %, along the longitudinal axis of the injector tip. Thus, a lateral curve geometry can be provided over the entire path through the injector tip in the guide channel, which only relatively slightly varies such that a folding operation for the intraocular lens is allowed over the entire path through the guide channel, which accomplishes the lateral upwards guidance and folding of the exterior regions of the intraocular lens in mechanically very gentle manner. Moreover, by such a symmetric and shape-specific configuration of the lateral curves, a corresponding movement clearance for the regions of the intraocular lens to be folded is also provided. Here too, the lens is guided in the guide channel in not exactly fitting manner, but here too, a corresponding clearance to the top is present such that certain deviation of the lens is also ensured in the folding operation.

Preferably, a length of a connecting straight line between a minimum of the top contour constituting a first end of the top curve and a second end of the top curve is between 55 % and 90 % of the length of a horizontal straight line between a vertical axis of symmetry of the guide channel and a maximum of a lateral curve. By such a configuration, in particular, it is ensured that a top curve is virtually relatively flat compared to the entire height of the internal space, however, preferably has a relatively great width defined by the horizontal connecting straight line of the endpoints on the other hand. By such a configuration of a top curve compared to the entire internal space geometry of the guide channel in cross-section, in particular in the second half, here too, geometric configurations are provided, which ensure particularly gentle folding of the lens, yet the desired folding direction is reliably and securely achieved.

Preferably, viewed in a cross-sectional representation, the guide channel and in particular the contour development thereof is formed symmetrically to this vertical axis of symmetry over the entire length. This means that the top curves and lateral curves opposing the axis of symmetry are formed in analog manner. Correspondingly, the bottom curve is also formed symmetrically to this vertical axis of symmetry.

Preferably, it is provided that a horizontal connecting straight line between two endpoints of a top curve viewed in the second half of the guide channel is greater than a vertical distance between the maximum of the top curve and the horizontal connecting straight line by a factor greater than or equal to 2. By this specific geometric specification, the very flat yet widely extending contour of the top curve is augmented in particular in the second half of the guide channel such that a particularly mechanically unstressed folding of the lens can be ensured in particular upon entrance of the intraocular lens from a receiving space into the injector tip.

In particular, in the context of the invention, it is suggested that substantially the entire folding operation of the lens is not performed in a cassette and thus not in one of the regions of the injector device preceding the injector tip, but that at best a slight bending of the lens is performed in front of the injector tip and then substantially the entire folding operation is performed in the injector tip itself.

In particular, thus, thereby too, the advantage is achieved that the lens can also be advanced into the injector tip relatively unfolded and can be folded there via a relatively long internal space compared to the receiving space in continuous and more gentle manner, and then, at the exit of the injector tip, the preferred and desired folded shape is present, which then is also permanently retained there. A ball formation or plug formation of a greatly pre-folded lens upon introduction from the receiving space into the injector tip can thereby be prevented.

Preferably, in the second half of the guide channel viewed along the longitudinal axis, at least in sections, a vertical distance between a minimum of the bottom curve and a minimum of the top contour between the two top curves is greater, in particular greater by at least a factor of 5, than a vertical distance between the maximum of a top curve and a horizontal connecting straight line through the minimum of the top contour. By this geometric specification too, in further possibility of specification, a limitation of the guide channel is provided, by which a corresponding clearance for the lens to the top is present, and moreover, a particularly unstressed bending and folding of lateral regions of the lens can be ensured by the relatively great and flat top curve geometry.

In particular in this rear second half of the injector tip, by the invention or an advantageous development thereof, a mechanically very gentle folding operation is initiated and performed, which considerably reduces the mechanic stresses by bending and folding with respect to the prior art and then allows in the first half of the guide channel to complete the transfer of this intermediate folded state into the final folded state in similar manner with considerably reduced mechanical stress potential for the lens. Thus, by the specific cross-sectional shapings of the guide channel considered over the entire guide channel, a particularly adapted and very harmonic folding with regard to reliable folding direction and reduced mechanical stress of the lens is ensured, and moreover, a particularly advantageous final folded position at the exit of the injector tip is provided and retained.

Preferably, it is provided that at least in the second half a length ratio between a vertical connecting straight line connecting the endpoints of a lateral curve and a vertical distance between a maximum of a top curve and a minimum of the bottom curve is between 0.6 and 0.8, in particular between 0.65 and 0.78. This is an advantageous configuration in particular to be emphasized geometrically, because this ratio formation between the geometry and the size of a lateral curve and a subsequent top curve allows a particularly continuous folding operation with relatively uniform mechanical force effects over the entire region of the lens to be folded, such that undesirably great force effects at small specific locations can be avoided.

Preferably, the lateral curves and the top curves in the second half of the guide channel are formed such that a height distance between the bottom and a contour depression of the top contour, in particular a minimum in the top contour, between the two top curves is at least 10 %, in particular at least 20 %, greater than a vertical thickness of the intraocular lens to be transported in the guide channel in this position of the contour depression on the axis of symmetry. By this configuration, a particularly suitable internal space relationship of the guide channel is provided in the second half, since thus, in particular at this rear section of the guide channel, relatively much clearance to the top is provided for the lens, and it has a corresponding distance to the top contour. Thereby, the initiation of the folding operation can be effected in particularly advantageous manner and notably without great force effects on the lens.

Not least, such a configuration in this respect also ensures a particular advantage with regard to the folding geometry then finally to be achieved in the first half with the lateral edges of the lens facing the top side.

Preferably, the radius of a top curve is formed decreasing, in particular formed continuously decreasing, from a first to a second end viewed along the longitudinal axis of the injector tip. This allows a geometric configuration with a further advantageous influence of the particularly gentle folding operation and the final folded position to be achieved. Thus, in the top region with more and more decreasing radius, the lens is guided with its lateral edges towards the top of the lens in particularly specific and directed manner.

Preferably, the radius of a lateral curve is formed increasing, in particular formed continuously increasing, from a first to a second end viewed along the longitudinal axis of the injector tip. The advantages mentioned in the preceding paragraph with regard to the radius formation of the top curve apply here to the radius formation of the lateral curve in analog manner, and in particular in conjunction with the above-mentioned advantageous configuration, this is to be particularly emphasized.

It can be provided that the radius of a lateral curve is constant at a cross-sectional location considered over the entire curve length. It can also be provided that the radius varies at a cross-sectional location over the curve length. The same can also be formed for the radius formation of the top curve.

Preferably, a radius of a top curve in the second half of the guide channel of the injector tip is formed between 50 % and 150 % of a radius of a lateral curve.

It can be provided that the radius of a lateral curve viewed over the entire length of the guide channel is greater than or equal to 0.6 mm, in particular between 0.7 mm and 1 mm. By this configuration, thus, relatively large lateral curves are formed such that the initiation of the folding operation in particular in the second half of the guide channel is effected in particularly gentle manner and with particularly reduced mechanical influence on the lens.

Preferably, at least in the second half of the guide channel, the radius of a top curve is greater than 0.6 mm, in particular between 0.6 mm and 0.8 mm. Here too, the above-mentioned advantages apply in analog manner.

Preferably, the guide channel of the injector tip has a shaping confining the internal space of the channel, by which an intraocular lens is folded at least at a location in a front first half viewed along the longitudinal axis of the guide channel such that opposing lateral edges of the intraocular lens rest on the top side of the optic part of the intraocular lens. Thus, preferably, a direct contact between the lateral edges and the top side of the optic part is caused in the folding operation. This ensures particularly harmonic folded shape with regard to the mechanical influence of the folded regions of the lens and moreover forms a particularly compact folded shape of the lens.

Preferably, a third outwardly curved top curve is formed between the two top curves at least in sections in the second half of the guide channel, which has a smaller curve length and a shorter horizontal connecting straight line compared to the two first top curves between its endpoints, wherein these mentioned geometric parameters are in particular multiple times smaller than the geometric parameters of the two first top curves. Preferably, this third top curve is formed for guiding a plunger for advancing the lens out of the injector tip.

Preferably, the injector tip is formed for connecting to a cassette, in which the intraocular lens is received in an injector device in its rest position and from which the lens can be advanced from the receiving space into the injector tip by means of a plunger. Thus, it is in particular provided that this receiving space for the intraocular lens in its rest position is not a component of the injector tip and this receiving space is virtually disposed in front of the injector tip viewed in a longitudinal direction of an injector device. The injector tip can be designed as a separate part to that, in which the receiving space for the intraocular lens in its rest position is formed. However, it can also be provided that the injector tip is formed integrally with this mentioned component.

The geometric specifications and designations between the connecting straight lines are in particular to be taken as a basis such that they extend in the guide channel and only intersect the contour of the shaped wall at the ends of the curves. Furthermore, in particular, the connecting straight lines are to be understood such that a horizontal and a vertical connecting straight line intersect on the contour. Correspondingly, thereby, the top curves, the lateral curves and the bottom curve are also geometrically defined. In particular, a curved line is understood as a curve, which has a curving direction over its entire curve length.

Furthermore, the invention relates to an injector device with an injector tip according to the first aspect of the invention or an advantageous development thereof or with an injector tip according to the second aspect of the invention or an advantageous development thereof.

In particular, the injector device includes an injector tube, on which the injector tip is disposed at the front end, and in the injector tube, in particular in a cassette attachable in the injector tube in front of the injector tip, a receiving space for the intraocular lens is formed, wherein the receiving space is formed tapered, in particular continuously tapered, towards the injector tip in a longitudinal section view. The intraocular lens is received in its rest position in this receiving space, wherein it is pre-folded to some degree by the tapered configuration of the receiving space upon advancing out of the receiving space by means of the plunger, wherein this stop operation only effects slight bending of the lens. By this configuration, a particularly gentle and continuous transition into the injector tip and the guide channel is achieved such that a particularly gentle transition for further performing the folding in the injector tip is ensured. Moreover, by this configuration of the receiving space, an abrupt transition and an abrupt cross-sectional reduction from the receiving space into the guide channel can be avoided such that exactly the problems mentioned to the prior art can here be avoided.

Preferably, the receiving space has confining walls formed such that the intraocular lens is disposed inclined to the bottom in its rest position in the receiving space, wherein a ceiling wall of the receiving space has a guide region for a plunger for advancing the lens out of the receiving space into the injector tip, and the plunger is bent downwards by the guide region upon movement into the receiving space. Thus, a guide for the plunger is formed integrally in a confining wall of the receiving space at the same time, which specifically does not remain in its linear movement along its longitudinal axis upon advancing the lens out, but is also guided downwards to some degree. Such a specific rest position of the intraocular lens with such a path for advancing-out specific in this respect and the bending of the plunger allows pre-folding of the lens in the receiving space in particularly simple and low-wear manner on the one hand, and moreover, a particularly advantageous continuous transfer into the guide channel in the injector tip is allowed.

Further features of the invention are apparent from the claims, the figures and the description of figures.

### Brief description of the drawings

Embodiments of the invention are explained below in more detail by way of schematic drawings. There show:
- Fig. 1: a schematic perspective representation of an embodiment of an injector device according to the invention;
- Fig. 2: a perspective representation of an embodiment of an injector tip according to the invention;
- Fig. 3: a rear view of the injector tip according to fig. 2;
- Fig. 4: an enlarged representation of a partial region of the injector device according to fig. 1;
- Fig. 5: a first sectional representation of the injector device according to fig. 4;
- Fig. 6: a second sectional representation of the injector device according to fig. 4;
- Fig. 7: a perspective view from the receiving space of the injector device into the injector tip;
- Fig. 8: a sectional representation through the receiving space with an intraocular lens disposed in the rest position;
- Fig. 9: a further sectional representation of the injector device according to fig. 4 analog to the sectional representation according to fig. 6;
- Fig. 10a and 10b: cross-sectional representations of the guide channel in a second half along the longitudinal axis without inserted intraocular lens and with inserted intraocular lens, respectively;
- Fig. 11a and 11b: further cross-sectional representations of the guide channel in a second half along the longitudinal axis without inserted intraocular lens and with inserted intraocular lens, respectively;
- Fig. 12a and 12b: a further cross-sectional representation of the guide channel in a front first half viewed along the longitudinal axis of the guide channel without inserted intraocular lens and with inserted intraocular lens, respectively; and
- Fig. 13a and 13b: a further cross-sectional representation of the guide channel in a front first half viewed along the longitudinal axis of the guide channel without inserted intraocular lens and with inserted intraocular lens, respectively.

### Preferred embodiments of the invention

In the figures, similar or functionally equivalent elements are provided with the same reference characters.

In fig. 1, in a perspective representation, an embodiment of an injector device 1 according to the invention is shown. By means of this injector device, an intraocular lens can be inserted into the eye. The injector device 1 includes an injector tube 2, in which a not shown plunger is displaceable in x-direction. An intraocular lens inserted in a cassette 3 in a receiving space formed therein can be advanced out by the plunger. It can be provided that the cassette 3 can be detachably inserted into a frame 2a and is formed as a separate part to the injector tube 2 and also as a separate part to an injector tip 4. The injector tip 4 joins to the frame 2a at the front end. The injector tip 4 can be integrally connected to the frame 2 and thus also to the injector tube 2. In particular, it is provided that the injector tip 4 can be attached to the frame 2a in detachable and thus in non-destructive manner and is disposed thereon separable again.

In fig. 2, in a perspective representation, an embodiment of an injector tip 4 according to the invention is shown. The injector tip 4 includes a rear end 5, which can be connected to the frame 2a. Moreover, the injector tip 4 includes a front end 6, from which an intraocular lens can be advanced out of the injector device 1 and can be inserted into an eye. In this respect, the front end 6 is introduced into the eye. It is appreciable that the exterior shaping of the injector tip 4 tapers from the rear end 5 to the front end 6 such that the front end 6 is relatively small dimensioned in order to be able to be introduced in a small incision in the eye.

With regard to the later following explanations, the injector tip 4 is divided into a front first half I and a rear second half II directly adjoining thereto. The injector tip 4 has a longitudinal axis A extending in x-direction in the illustration according to fig. 1.

In fig. 3, a rear view of the injector tip 4 and thus to the rear end 5 is shown. In the interior of the injector tip 4, a guide channel 7 is formed, which represents a cavity or an internal space confined by shaped walls 8. It is appreciable that the cross-sectional area at the rear end 5 is substantially greater than the cross-sectional area at the front end 6. The shaped wall 8 is also designed to the effect that it is formed tapered from the rear end 5 to the front end 6 viewed along the longitudinal axis A. As it is apparent from the illustration in fig. 3, the guide channel 7 has a bean-like shaping in cross-section in the region of the rear end 5, in particular at least in the second half II.

The injector tip 4 is formed with its guide channel 7 such that substantially the entire folding operation of the intraocular lens is effected in the injector tip 4, and in particular at the front end 6, a folded shape of the intraocular lens viewed in cross-section is provided, in which exterior lateral edges of the intraocular lens rest on a top side of the optic part of the intraocular lens.

In fig. 4, in a perspective representation, a front region of the injector device 1 according to fig. 1 is shown.

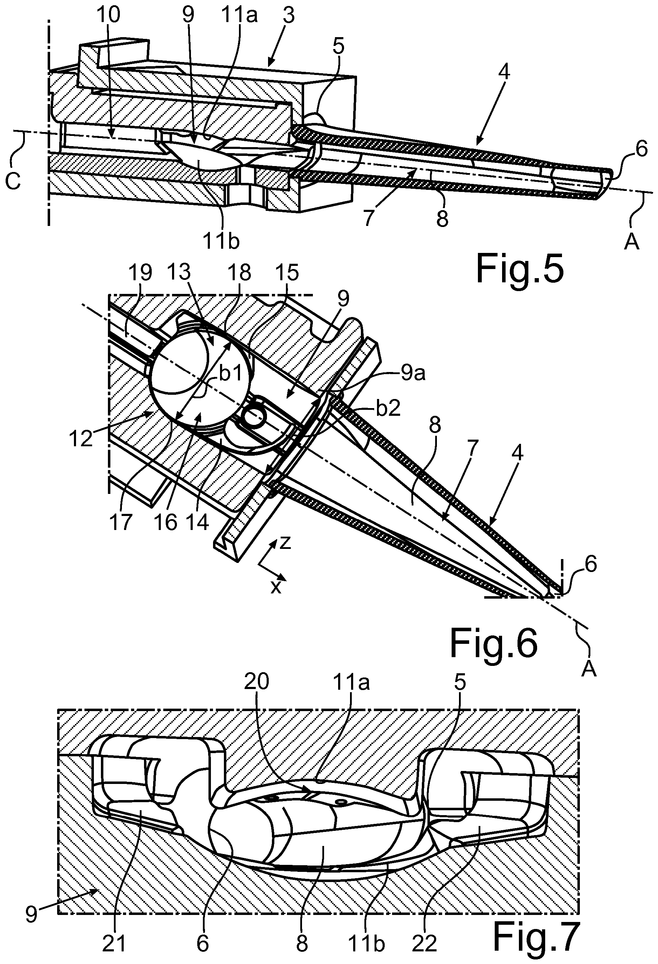

In fig. 5, a first sectional representation of fig. 4 is shown, wherein a cross-sectional representation in the x-y-plane along the sectional line V-V is shown in this respect. In this sectional representation, the receiving space 9 for the intraocular lens is illustrated. It has a channel 10, in which the plunger is guided. Moreover, the receiving space 9 includes a ceiling 11a having an integrated guide for the plunger. Moreover, a bottom 11b is formed on the opposing side of the ceiling 11a. The ceiling 11a and the bottom 11b are formed outwardly curved and moreover oriented such that the intraocular lens inserted therein in its rest position is inclined to the bottom with respect to the horizontal and thus with respect to the x-axis. This means that it is disposed with its front side facing the injector tip 4 located lower than with its side facing the channel 10.

By the ceiling 11a with its integrated guide for the plunger, the plunger arriving through the channel 10 is contacted with the intraocular lens, and it is advanced out of the receiving space 9, wherein the plunger then is virtually pressed downwards and thus bent downwards by the guide in the ceiling 11a in the receiving space 9. In the further progress, the intraocular lens exits the receiving space 9 and enters the guide channel 7 of the injector tip 4, where it is advanced through the plunger up to the front end 6 afterwards.

In fig. 6, a further sectional representation along the sectional line VI-VI in fig. 4 is shown. A sectional plane in the x-z-plane is illustrated in this respect, and fig. 6 shows a top view of the cut-open injector device 1. In this configuration, an intraocular lens 12 is exemplarily received in the receiving space 9, which includes a circular optic part 13, on which a first haptic part 14 and a second haptic part 15 are disposed. The intraocular lens 12 shown here has hook-shaped or C-shaped haptic parts 14 and 15. In particular, the intraocular lens 12 is formed as an asymmetric lens, which means that opposing surfaces and thus a top side and a bottom side of the optic part 13 have different curvatures.

In the shown representation, the lens 12 is illustrated with its top side 16 in its rest position in the receiving space 9. This rest position is the inclined base position already mentioned to fig. 5, wherein the lens 12 moreover is disposed in the relaxed state. Moreover, it is disposed in a free state in the receiving space 9, which means that it is positioned virtually floating and non-contacting with respect to its optic part 13 to the confining walls of the receiving space 9. In particular, a liquid can be introduced in the receiving space 9 in this respect, thereby retaining this floating state. Moreover, in fig. 6, the plunger 19 is shown as it is in contact with a lateral edge of the optic part 13. The lens 12 and in particular the optic part 13 has lateral edges 17 and 18, by which the top side 16 and the bottom side are connected. In the embodiment, the diameter of the lens 12 and in particular of the optic part 13 is dimensioned slightly smaller than an internal dimension b1 of the opposing sidewalls of the receiving space 9. In this respect, this distance b1 relates to that in the x-z-plane.

As it is apparent in the representation according to fig. 6, at its front end 9a, the receiving space 9 has a width and thus a distance b2 between the opposing confining walls, which is smaller than the distance b1. This means, that the receiving space 9 tapers viewed with respect to its width towards the injector tip 4 and thus towards the front end 9a. Thereby, the lens 12 is at least slightly pre-folded upon advancing along in the receiving space 9 such that it experiences a certain upwards bending of the lateral edges 17 and 18.

In fig. 7, a further perspective representation is shown, wherein a view from the receiving space 9 towards the injector tip 4 is illustrated in this respect. It is apparent that the guide 20 for the plunger 19 is formed in the outwardly curved ceiling wall 11a. Moreover, at the bottom 11b on lateral regions, bottom bulges 21 and 22 are formed, by which the upwardly bent pre-folding of the lens 12 is additionally favored before entering the injector tip 4.

In fig. 8, a sectional representation in the x-y-plane of a partial section of the injector device 1 is shown, wherein a sectional representation of the receiving space 9 with the intraocular lens 12 in its rest position is shown in this respect. The forward and downward inclined rest position is illustrated, wherein an angle α between the center plane B of the intraocular lens 12 and the longitudinal axis C of the plunger 19 or that of the injector tube 2 is shown.

Moreover, an angle β is formed between the center plane B and the longitudinal axis A of the injector tip 4. The angles α and β can be of equal size, however they can also be different.

In fig. 9, a further sectional representation in the x-z-plane is shown, which corresponds to the representation in fig. 6, wherein the lens 12 is removed in this respect. Moreover, in the injector tip 4, at different locations along the longitudinal axis A, sectional planes E, F, G, H are drawn. They are shown in specific distances 11, 12, 13 and 14 to the front end 9b of the frame 2a.

The cross-sectional representations of the guide channel 7 and thus also of the injector tip 4 on the sectional planes E, F thus show cross-sectional shapes of the guide channel 7 in the second half II, whereas the cross-sectional representations on the sectional planes G, H show such ones, which are formed in the first half I of the injector tip 4.

In the embodiment, the length 11 is 0.5 mm, the length 12 is 5 mm, the length 13 is 10 mm and the length 14 is 15 mm.

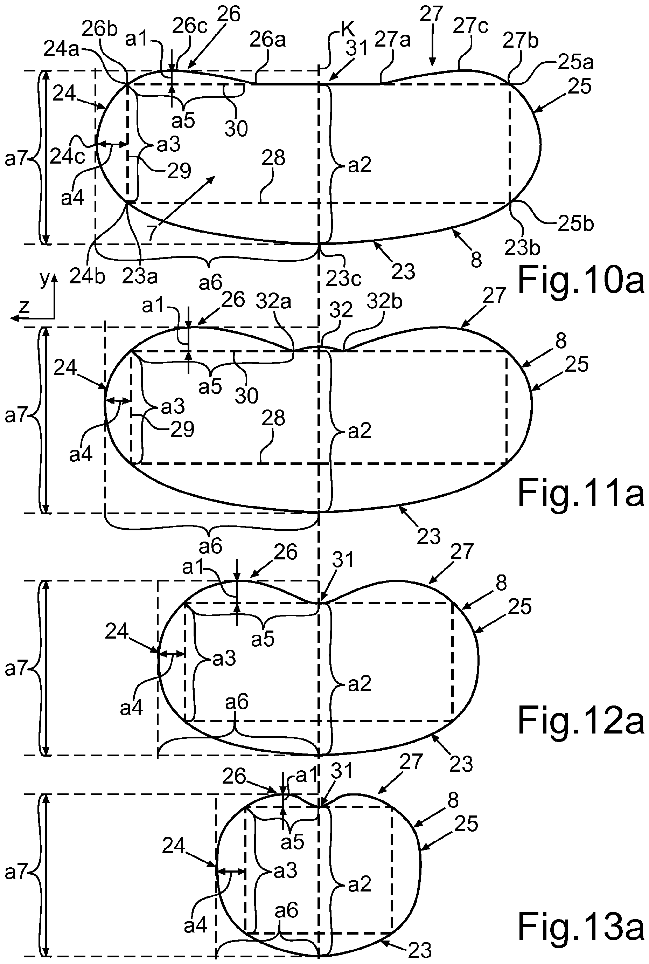

In the following figures 10a, 10b, 11a, 11b, 12a, 12b, 13a and 13b, these cross-sectional representations of the sectional planes E, F, G and H are shown once without inserted intraocular lens 12 and once with half-sided inserted intraocular lens 12.

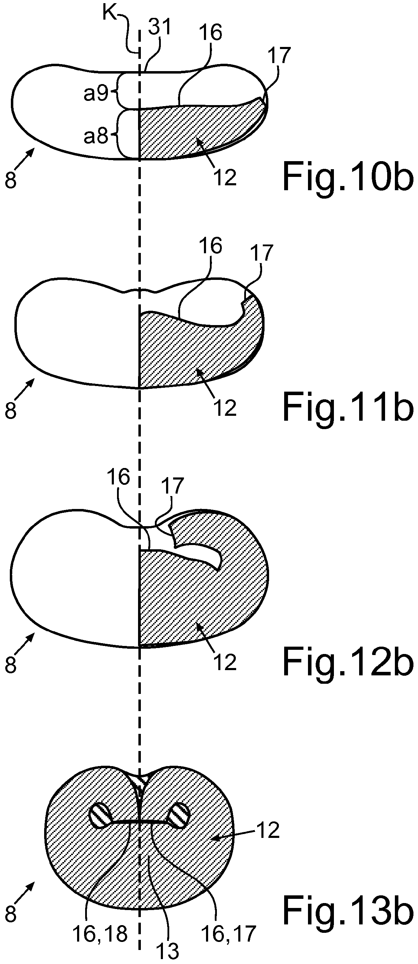

In fig. 10a and 10b, thus, the cross-section of the guide channel 7 with the shaped wall 8 is shown at the location of the sectional plane E.

It is apparent that the contour development of the shaped wall 8 has a bottom curve 23, which is arched outwardly and thus downwardly curved. The bottom curve 23 has a first end 23a and a second end 23b. The outward curvature in this respect is seen based on a view in the guide channel 7.

Subsequent to the bottom curve 23, a first lateral curve 24 is formed, which is also formed outwardly curved. The first lateral curve 24 includes a first end 24a and a second end 24b.

On the opposing side, a second lateral curve 25 is formed, which is formed analog to the first lateral curve 24 and has a first end 25a and a second end 25b. Subsequent to the first lateral curve 24, a first top curve 26 is formed, which has a first end 26a and a second end 26b. In corresponding manner, a second top curve 27 is formed, which has a first end 27a and a second end 27b.

The shaped wall 8 is formed symmetrically to a vertical axis of symmetry K over the entire length of the guide channel 7 along the axis A.

The top curves 26 and 27 are also formed outwardly curved.

According to the geometric definition taken as a basis, here, it is provided that the curves 23 to 27 are formed immediately adjoining each other such that an end of a first curve represents the beginning of the other curve. Moreover, in the embodiment, it is provided that all of the curves 23 to 27 have a similar curving direction over their curve lengths.

Moreover, a horizontal connecting straight line 28 is drawn, which connects the two endpoints 23a and 23b of the bottom curve 23 and extends in the space of the guide channel 7 and thus only has intersections with the contour of the shaped wall 8 at these ends 23a and 23b. Moreover, a vertical connecting straight line 29 is drawn, which connects the endpoints 24a and 24b of the first lateral curve 24. It is vertically oriented and intersects the contour development of the shaped wall 8 also merely at these two ends 24a and 24b, wherein the intersection between the two connecting straight lines 28 and 29 is effected on the shaped wall 8 in this respect and thus is present in the region of the endpoints 23a and 24b. In corresponding manner, a vertical connecting straight line of the lateral curve 25 not identified in more detail is drawn between the endpoints 25a and 25b.

Moreover, a horizontal connecting straight line 30 is drawn, which connects the two endpoints 26a and 26b of the top curve 26. It intersects with the vertical connecting straight line 29 on the contour development and thus on the shaped wall 8 at the location of the endpoints 24a and 26b.

Moreover, the endpoint 26a of the top curve 26 is defined such that it represents the lowest location of the top contour, wherein the top contour results from the top curves 26 and 27 and a contour depression 31, which represents a horizontal straight line between the endpoints 26a and 27a in the cross-sectional representation according to fig. 10a. Thus, the endpoints 26a and 27a are a minimum of the top contour at the same time.

Moreover, the top curve 26 has a maximum 26c and the top curve 27 has a maximum 27c. Thus, the maximum 26c has a greatest distance a1 between the top curve 26 and the connecting straight line 30. A distance between the horizontal connecting straight line not identified in more detail between the endpoints 27a and 27b of the top curve 27 to the maximum 27c is correspondingly dimensioned.

This distance a1 is multiple times smaller than a vertical distance a2 between a contour depression in the top contour and a minimum 23c of the bottom curve 23. In the shown implementation, the distance a2 is greater than the distance a1 by at least a factor of 5.

Moreover, in the shown implementation, the sum of the distances a1 and a2 is equal to the maximum external dimension a7 of the shaped wall 8 in vertical direction, which extends between the maximum 26c or 27c and the minimum 23c projected onto the axis of symmetry K.

The vertical connecting straight line 29 has a height a3. Moreover, a distance a4 is formed between the connecting straight line 29 and the maximum 24c. A horizontal distance a5 is formed between the endpoints 26a and 26b, wherein the length of the connecting straight line 30 is given thereby.

In analog manner, the shaped wall 8 is formed on the opposing side with the lateral curves 25 and the top curve 27.

In particular, a lateral curve 24 or 25 and a top curve 26 or 27 adjoining thereto are formed such that a length ratio between an associated vertical connecting straight line 29 and the adjacent connecting straight line 30 of the connecting straight lines 29 and 30 intersecting on the contour development is between 0.7 and 0.95 in the second half II of the guide channel 7.

Moreover, the guide channel 7 has a shaped wall 8, by which the intraocular lens 12 is folded at least at a location in a front first half I viewed along the longitudinal axis A of the guide channel 7 such that opposing lateral edges 17 and 18 face a top side 16 of the optic part 13 of the intraocular lens 12, in particular rest thereon.

According to the representation in fig. 10a and 10b, the lateral curves 24 and 25 are formed at least in the second half II such that a length or height a3 of a vertical connecting straight line 29 along the longitudinal axis A of the injector tip 4 is varied by a maximum of 20%, in particular a maximum of 10 %.

Moreover, it is provided that a length a5 of a horizontal connecting straight line 30 is between 55 % and 90 % of a horizontal linear distance a6 between the vertical axis of symmetry K of the guide channel 7 from a maximum 24c of a lateral curve 24.

Moreover, the shaped wall 8 is formed in the second half II such that a connecting straight line 30 of a top curve 26 is greater than the distance a1 by a factor greater than or equal to 2. In the representation according to fig. 10a, this distance a5 is at least five times greater than the distance a1.

Preferably, at least in the second half II, a length ratio of the length a3 of the vertical connecting straight line 29 to the distance a7, which is the sum of the distances a1 and a2 in the shown implementation and thus shows the maximum height extension of the shaped wall 8, is between 0.6 and 0.8, in particular between 0.65 and 0.78.

In particular, the lateral curves 24 and 25 and the top curves 26 and 27 are formed in the second half II of the guide channel 7 such that a distance a2 between the bottom curve 23, in particular the minimum 23c, and the contour depression, in particular a minimum 26a or 27a of the top contour, is at least 10 %, in particular at least 20 %, greater than a vertical thickness a8 (fig. 10b) of the intraocular lens 12 to be transported in the guide channel 7 in this position and thus in the region of the axis of symmetry K.

In particular, a radius of a top curve 26 or 27 viewed along the longitudinal axis A of the injector tip 4 is formed decreasing, in particular formed continuously decreasing, from the rear end 5 towards the front end 6. In particular, the radius of a lateral curve 24 or 25 viewed along the longitudinal axis A is also formed increasing, in particular formed continuously increasing, from the rear end 5 to the front end 6 of the injector tip 4. In particular, the radius of a top curve is greater than 0.6 mm, in particular between 0.6 mm and 0.8 mm, at least in the second half II of the guide channel 7. Moreover, the radius of a lateral curve 24 or 25 is greater than or equal to 0.6 mm, in particular between 0.7 mm and 1 mm, in particular viewed over the entire length of the guide channel 7.

With regard to the definitions shown in fig. 10a in enlarged representation, the curves are to be understood such that they all directly adjoin to each other, wherein the contour depression 31 is formed between the top curves. Moreover, the horizontal and vertical connecting straight lines 28, 29 and 30 are formed extending in the internal space and drawn intersecting on the contour development and thus on the shaped wall 8 in pairs and to be understood geometrically.

In particular with regard to that and the geometric specifications drawn in fig. 10a to 13a, with regard to the shaping, arrangement, orientation and finally entire configuration of the guide channel 7 with its shaped wall 8 are to be understood.

In the representation according to fig. 10b, moreover, a distance a9 is shown, which shows the distance between the top side 16 of the lens 12 and the contour depression 31. It is appreciable that this distance is relatively great and can be up to 80 % of the thickness a8. As is apparent, thus, the lens 12 is formed with a considerable clearance and movement space in vertical direction with regard to the configuration of the shaped wall 8 and not inserted therein fitting nearly exactly, as it is the case in the prior art.

In fig. 11a and 11b, the cross-section on the sectional plane F is shown. In this respect, a third outwardly curved top curve 32 with ends 32a and 32b is illustrated in the top contour, wherein it is substantially smaller, which means that it is formed with a substantially lower height and a substantially lower width than the two first top curves 26 and 27.

With regard to the configurations in the fig. 12a and 13a, the cross-sections on the sectional planes G and H are shown in this respect. Based on the illustration in fig. 10a, it is appreciable that the horizontal width of the guide channel 7 considerably decreases up to the illustration in fig. 13a, wherein the height is substantially slightly varied.

Moreover, it is to be mentioned that the length a3 of the connecting straight line 29 varies by a maximum of 20 %, in particular a maximum of 10 %, starting from the illustration in fig. 10a to the illustration in fig. 13a.

According to the illustration in fig. 10a to 13a, it is apparent that the contour development is formed with relatively great lateral curves 24 and 25 and moreover relatively wide top curves 26 and 27.

By the previously explained geometric ratios both in the cross-sectional representations and along the longitudinal axis A in the first half I and the second half II, a particularly gentle and continuous folding operation of the lens 12 is achieved without subjecting it to undesirably great mechanical stresses, in particular local great mechanical stresses.

In the representations according to 10b to 13b, the folding of the lens is apparent, wherein only a slight elevation of the lateral regions of the lens 12 has been effected starting from the representation in fig. 10b. In the further steps, then, a gentle and uniform upward bending of the exterior regions of the lens 12 and thus also of the lateral edges 17 and 18 is successively and continuously performed due to the large curve width of the lateral curves 24 and 25. By the guide channel 7 being formed relatively high (extension in y-direction) and having a corresponding clearance, the folding can be effected very harmonically afterwards according to the illustrations in fig. 12b and 13b. This terminates in the illustration according to fig. 13b in that the lateral edges 17 and 18 directly rest on the top side 16 of the optic part 13, which in particular results from the top curves 26, 27. The folded external regions of the lens 12 are, as is shown in fig. 13b, curved in virtually uniform curve shape. An undesirably intense flat-pressing of these folded regions towards the optic part is thus avoided and the folded external regions of the lens 12 thus describe a relatively large and high curve in the cross-sectional representation.

## Claims

1. Injector tip for an injector device (1) for introducing an intraocular lens (12) into an eye, which has a continuous guide channel (7) for the intraocular lens (12),
**characterized in that**
in cross-section, the guide channel (7) has at least in sections along a rear second half (II) a contour development with at least two outwardly curved top curves (26, 27), to which each two outwardly curved lateral curves (24, 25) join, which are connected by an outwardly curved bottom curve (23), wherein a lateral curve (24, 25) and a top curve (26, 27) are formed such that at least in the second half (II), a length ratio between a vertical connecting straight line (29) extending between two endpoints (24a, 24b; 25a, 25b) of a lateral curve (24, 25), and a horizontal connecting straight line (30) extending between a minimum of the top contour constituting a first end (26a, 27a) of a top curve (26, 27) and a second end (26b, 27b) of the top curve (26, 27), is formed between 0.7 and 0.95.

2. Injector tip according to claim 1,
**characterized in that**
the guide channel (7) has a shaped wall (8) confining the internal space of the channel (7), by which the intraocular lens (12) is folded at least at a location in a front first half (I) viewed along the longitudinal axis (A) of the guide channel (7) such that opposing lateral edges (17, 18) of the intraocular lens (12) are turned towards a top side (16) of an optic part (13) of the intraocular lens (12).

3. Injector tip according to anyone of the preceding claims,
**characterized in that**
the lateral curves (24, 25) are formed at least in the second half (II) such that a length of a vertical connecting straight line (29) between the two endpoints (24a, 24; 25a, 25b) of a lateral curve (24, 25) along the longitudinal axis (A) of the injector tip (4) is varied by a maximum of 20%, in particular a maximum of 10%.

4. Injector tip according to anyone of the preceding claims,
**characterized in that**
at least in the second half (II), a length (a5) of a connecting straight line (30) between a minimum of the top contour constituting a first end (26a, 27a) of a top curve (26, 27) and a second end (26b, 27b) of the top curve (26, 27) is between 55 % and 90% of the length (a6) of a horizontal straight line between a vertical axis of symmetry (K) of the guide channel (7) and a maximum (24c, 25c) of a lateral curve (24, 25), and/or a horizontal connecting straight line (30) between two endpoints (26a, 26b; 27a, 27b) of a top curve (26, 27) in the second half (II) is greater than a vertical distance (a1) between the maximum (26c, 27c) of a top curve (26, 27) and the horizontal connecting straight line (30) by a factor greater than or equal to 2.

5. Injector tip according to anyone of the preceding claims,
**characterized in that**
in the second half (II) at least in sections, a vertical distance (a2) between a minimum (23c) of the bottom curve (23) and a minimum (26a, 27a; 31) of the top contour between the two top curves (26, 27) is greater, in particular greater by at least a factor of 5, than a vertical distance (a1) between the maximum (26c, 27c) of a top curve (26, 27) and a horizontal connecting straight line (30) through the minimum (26a, 27a; 31) of the top contour.

6. Injector tip according to anyone of the preceding claims,
**characterized in that**
at least in the second half (II), a length ratio between a vertical connecting straight line (29) connecting the endpoints (24a, 24b; 25a, 25b) of a lateral curve (24, 25) and a vertical distance (a7) between a maximum (26c; 27c) of a top curve (26, 27) and a minimum (23c) of the bottom curve (23) is between 0.6 and 0.8, in particular between 0.65 and 0.78.

7. Injector tip according to anyone of the preceding claims,
**characterized in that**
the lateral curves (24, 25) and the top curves (26, 27) in the second half (II) of the guide channel (7) are formed such that a distance (a2) between the bottom minimum (23c) and a contour depression (31), in particular a minimum (26a, 27a) in the top contour, between the two top curves (26, 27) is at least 10%, in particular at least 20%, greater than a vertical thickness (a8) of the intraocular lens (12) to be transported in the guide channel in this position of the contour depression (31).

8. Injector tip according to anyone of the preceding claims,
**characterized in that**
the radius of a top curve (26, 27) from a first (26a, 27a) to a second end (26b, 27b) viewed along the longitudinal axis (A) of the injector tip (4) is formed decreasing, in particular formed continuously decreasing, and/or the radius of a lateral curve (24, 25) from a first (24a, 25a) to a second end (24b, 25b) viewed along the longitudinal axis (A) of the injector tip (4) is formed increasing, in particular formed continuously increasing and/or a radius of a top curve (26, 27) in the second half (II) of the injector tip (4) viewed along the longitudinal axis (A) is formed between 50% and 150% of a radius of a lateral curve (24, 25) .

9. Injector tip according to anyone of the preceding claims,
**characterized in that**
the radius of a lateral curve (24, 25) viewed over the entire length of the guide channel (7) is greater than or equal to 0.6 mm, in particular between 0.7 mm and 1 mm and/or the radius of a top curve (26, 27) at least in the second half (II) of the guide channel (7) is greater than 0.6 mm, in particular between 0.6 mm and 0.8 mm.

10. Injector tip according to anyone of the preceding claims,
**characterized in that**
the guide channel (7) has a shaped wall (8) confining the internal space of the channel (7), by which an intraocular lens (12) is folded at least at a location in a front first half (I) viewed along the longitudinal axis (A) of the guide channel (7) such that opposing lateral edges (17, 18) of the intraocular lens (12) rest on the top side (16) of the optic part (13) of the intraocular lens (12).

11. Injector device with an injector tip (4) according to anyone of the preceding claims.

12. Injector device according to claim 11,
**characterized in that**
it has an injector tube (2), on which the injector tip (4) is disposed at the front end (9a), and in the injector tube (2), in particular in a cassette (3), which can be attached in the injector tube (2) in front of the injector tip (4), a receiving space (9) for the intraocular lens (12) is formed, wherein the receiving space (9) is formed tapered, in particular continuously tapered, towards the injector tip (4) in a longitudinal section view and/or the receiving space (9) has confining walls (11a, 11b), which are formed such that the intraocular lens (12) is disposed inclined to the bottom in the receiving space (9) in its rest position, wherein a ceiling wall (11a) of the receiving space (9) has a guide region (20) for a plunger (9) for advancing the lens (12) out of the receiving space (9) into the injector tip (4), and the plunger (19) is bent downwards by the guide region (20) upon movement into the receiving space (9) .

## Patentansprüche

1. Injektorspitze für eine Injektorvorrichtung (1) zum Einführen einer Intraokularlinse (12) in ein Auge, welche einen durchgehenden Führungskanal (7) für die Intraokularlinse (12) aufweist,
**dadurch gekennzeichnet, dass**
der Führungskanal (7) im Querschnitt zumindest abschnittweise entlang einer hinteren zweiten Hälfte (II) einen Konturenverlauf mit zumindest zwei nach außen gekrümmten Dachbögen (26, 27), an die jeweils zwei nach außen gekrümmte Seitenbögen (24, 25) anschließen, die durch einen nach außen gekrümmten Bodenbogen (23) verbunden sind, aufweist, wobei ein Seitenbogen (24, 25) und ein Dachbogen (26, 27) so ausgebildet sind, dass zumindest in der zweiten Hälfte (II) ein Längenverhältnis zwischen einer vertikalen Verbindungsgerade (29), die sich zwischen zwei Endpunkten (24a, 24b; 25a, 25b) eines Seitenbogens (24, 25) erstreckt, und einer horizontalen Verbindungsgerade (30), die sich zwischen einem ein erstes Ende (26a, 27a) eines Dachbogens (26, 27) bildenden Minimum der Dachkontur und einem zweiten Ende (26b, 27b) des Dachbogens (26, 27) erstreckt, zwischen 0,7 und 0,95 ausgebildet ist.

2. Injektorspitze nach Anspruch 1,
**dadurch gekennzeichnet, dass**
der Führungskanal (7) eine den Innenraum des Kanals (7) begrenzende Formwand (8) aufweist, durch welche die Intraokularlinse (12) zumindest an einer Stelle in einer entlang der Längsachse (A) des Führungskanals (7) betrachtet vorderen ersten Hälfte (I) so gefaltet ist, dass gegenüberliegende Seitenränder (17, 18) der Intraokularlinse (12) einer Oberseite (16) eines optischen Teils (13) der Intraokularlinse (12) zugewandt sind.

3. Injektorspitze nach einem der vorhergehenden Ansprüche,
**dadurch gekennzeichnet, dass**
die Seitenbögen (24, 25) zumindest in der zweiten Hälfte (II) so ausgebildet sind, dass eine Länge einer vertikalen Verbindungsgerade (29) zwischen den beiden Endpunkten (24a, 24; 25a, 25b) eines Seitenbogens (24, 25) entlang der Längsachse (A) der Injektorspitze (4) maximal 20%, insbesondere maximal 10% verändert ist.

4. Injektorspitze nach einem der vorhergehenden Ansprüche,
**dadurch gekennzeichnet, dass**
zumindest in der zweiten Hälfte (II) eine Länge (a5) einer Verbindungsgerade (30) zwischen einem ein erstes Ende(26a, 27a) eines Dachbogens (26, 27) bildenden Minimum der Dachkontur und einem zweiten Ende (26b, 27b) des Dachbogens (26, 27) zwischen 55 % und 90% der Länge (a6) einer horizontalen Geraden zwischen einer vertikalen Symmetrieachse (K) des Führungskanals (7) und einem Maximum (24c, 25c) eines Seitenbogens (24, 25) beträgt, und/oder eine horizontale Verbindungsgerade (30) zwischen zwei Endpunkten (26a, 26b; 27a, 27b) eines Dachbogens (26, 27) in der zweiten Hälfte (II) um einen Faktor größer oder gleich 2 größer als ein vertikaler Abstand (a1) zwischen dem Maximum (26c, 27c) eines Dachbogens (26, 27) und der horizontalen Verbindungsgerade (30) ist.

5. Injektorspitze nach einem der vorhergehenden Ansprüche,
**dadurch gekennzeichnet, dass**
in der zweiten Hälfte (II) zumindest abschnittweise ein vertikaler Abstand (a2) zwischen einem Minimum (23c) des Bodenbogens (23) und einem Minimum (26a, 27a; 31) der Dachkontur zwischen den beiden Dachbögen (26, 27) größer ist, insbesondere um mindestens einen Faktor 5 größer ist, als ein vertikaler Abstand (a1) zwischen dem Maximum (26c, 27c) eines Dachbogens (26, 27) und einer horizontalen Verbindungsgerade (30) durch das Minimum (26a, 27a; 31) der Dachkontur.

6. Injektorspitze nach einem der vorhergehenden Ansprüche,
**dadurch gekennzeichnet, dass**
zumindest in der zweiten Hälfte (II) ein Längenverhältnis zwischen einer die Endpunkte (24a, 24b; 25a, 25b) eines Seitenbogens (24, 25) verbindenden vertikalen Verbindungsgerade (29) und einem vertikalen Abstand (a7) zwischen einem Maximum (26c; 27c) eines Dachbogens (26, 27) und einem Minimum (23c) des Bodenbogens (23) zwischen 0,6 und 0,8, insbesondere zwischen 0,65 und 0,78 beträgt.

7. Injektorspitze nach einem der vorhergehenden Ansprüche,
**dadurch gekennzeichnet, dass**
die Seitenbögen (24, 25) und die Dachbögen (26, 27) in der zweiten Hälfte (II) des Führungskanals (7) so ausgebildet sind, dass ein Abstand (a2) zwischen dem Bodenminimum (23c) und einer Kontursenke (31), insbesondere ein Minimum (26a, 27a) in der Dachkontur, zwischen den beiden Dachbögen (26, 27) um zumindest 10%, insbesondere zumindest 20% größer als eine vertikale Dicke (a8) der in dem Führungskanal zu transportierenden Intraokularlinse (12) an dieser Lageposition der Kontursenke (31) ist.

8. Injektorspitze nach einem der vorhergehenden Ansprüche,
**dadurch gekennzeichnet, dass**
der Radius eines Dachbogens (26, 27) von einem ersten (26a, 27a) zu einem zweiten Ende (26b, 27b) entlang der Längsachse (A) der Injektorspitze (4) betrachtet sich verkleinernd ausgebildet ist, insbesondere sich kontinuierlich verkleinernd ausgebildet ist, und/oder der Radius eines Seitenbogens (24, 25) von einem ersten (24a, 25a) zu einem zweiten Ende (24b, 25b) entlang der Längsachse (A) der Injektorspitze (4) betrachtet sich vergrößernd ausgebildet ist, insbesondere sich kontinuierlich vergrößernd ausgebildet ist, und/oder ein Radius eines Dachbogens (26, 27) in der zweiten Hälfte (II) der Injektorspitze (4) entlang der Längsachse (A) betrachtet zwischen 50% und 150% eines Radius eines Seitenbogens (24, 25) ausgebildet ist.

9. Injektorspitze nach einem der vorhergehenden Ansprüche,
**dadurch gekennzeichnet, dass**
der Radius eines Seitenbogens (24, 25) über die gesamte Länge des Führungskanals (7) betrachtet größer oder gleich 0,6 mm, insbesondere zwischen 0,7 mm und 1 mm, beträgt, und/oder der Radius eines Dachbogens (26, 27) zumindest in der zweiten Hälfte (II) des Führungskanals (7) größer 0,6 mm, insbesondere zwischen 0,6 mm und 0,8 mm, beträgt.

10. Injektorspitze nach einem der vorhergehenden Ansprüche,
**dadurch gekennzeichnet, dass**
der Führungskanal (7) eine den Innenraum des Kanals (7) begrenzende Formwand (8) aufweist, durch welche eine Intraokularlinse (12) zumindest an einer Stelle in einer entlang der Längsachse (A) des Führungskanals (7) betrachtet vorderen ersten Hälfte (I) so gefaltet ist, dass gegenüberliegende Seitenränder (17, 18) der Intraokularlinse (12) auf der Oberseite (16) des optischen Teils (13) der Intraokularlinse (12) aufliegen.

11. Injektorvorrichtung mit einer Injektorspitze (4) nach einem der vorhergehenden Ansprüche.

12. Injektorvorrichtung nach Anspruch 11,
**dadurch gekennzeichnet, dass**
sie ein Injektorrohr (2) aufweist, an dem am vorderen Ende (9a) die Injektorspitze (4) angeordnet ist und in dem Injektorrohr (2), insbesondere in einer in das Injektorrohr (2) vor der Injektorspitze (4) anbringbaren Kassette (3), ein Aufnahmeraum (9) für die Intraokularlinse (12) ausgebildet ist, wobei der Aufnahmeraum (9) in einer Längsschnittbetrachtung zur Injektorspitze (4) hin verjüngt, insbesondere kontinuierlich verjüngt, ausgebildet ist, und/oder der Aufnahmeraum (9) Begrenzungswände (11a, 11b) auf-weist, die derart ausgebildet sind, dass die Intraokularlinse (12) in ihrer Ruheposition in dem Aufnahmeraum (9) nach unten geneigt angeordnet ist, wobei eine Deckenwand (11a) des Aufnahmeraums (9) einen Führungsbereich (20) für einen Kolben (19) zum Ausschieben der Linse (12) aus dem Aufnahmeraum (9) in die Injektorspitze (4) aufweist, und der Kolben (19) durch den Führungsbereich (20) beim Bewegen in dem Aufnahmeraum (9) nach unten gebogen ist.

## Revendications

1. Pointe d'injecteur pour un dispositif injecteur (1) pour introduire une lentille intraoculaire (12) dans un oeil, qui comporte un canal de guidage continu (7) pour la lentille intraoculaire (12),
**caractérisée en ce que**
en coupe transversale, le canal de guidage (7) comporte, au moins dans des sections le long d'un seconde moitié arrière (II), un développement de contour ayant au moins deux courbes supérieures courbées vers l'extérieur (26, 27), auxquelles chaque paire de courbes latérales courbées vers l'extérieur (24, 25) se rejoignent, qui sont raccordées par une courbe inférieure courbée vers l'extérieur (23), dans laquelle une courbe latérale (24, 25) et une courbe supérieure (26, 27) sont formées de telle sorte qu'au moins dans la seconde moitié (II), un rapport de longueur entre une ligne droite verticale de liaison (29) s'étendant entre deux points d'extrémité (24a, 24b ; 25a, 25b) d'une courbe latérale (24, 25) et une ligne droite horizontale de liaison (30) s'étendant entre un minimum du contour supérieur constituant une première extrémité (26a, 27a) d'une courbe supérieure (26, 27) et une seconde extrémité (26b, 27b) de la courbe supérieure (26, 27) soit formé entre 0,7 et 0,95.

2. Pointe d'injecteur selon la revendication 1, **caractérisée en ce que**
le canal de guidage (7) comporte une paroi façonnée (8) délimitant l'espace interne du canal (7), au moyen duquel la lentille intraoculaire (12) est pliée au moins à un endroit dans une première moitié avant (I) vue le long de l'axe longitudinal (A) du canal de guidage (7) de telle sorte que des bords latéraux opposés (17, 18) de la lentille intraoculaire (12) soient tournés vers un côté supérieur (16) d'une partie optique (13) de la lentille intraoculaire (12).

3. Pointe d'injecteur selon l'une quelconque des revendications précédentes,
**caractérisée en ce que**
les courbes latérales (24, 25) sont formées au moins dans la seconde moitié (II) de telle sorte qu'une longueur d'une ligne droite verticale de liaison (29) entre les deux points d'extrémité (24a, 24b ; 25a, 25b) d'une courbe latérale (24, 25) le long de l'axe longitudinal (A) de la pointe d'injecteur (4) soit modifiée d'un maximum de 20 %, en particulier d'un maximum de 10 %.

4. Pointe d'injecteur selon l'une quelconque des revendications précédentes,
**caractérisée en ce que**
au moins dans la seconde moitié (II), une longueur (a5) d'une ligne droite de liaison (30) entre un minimum du contour supérieur constituant une première extrémité (26a, 27a) d'une courbe supérieure (26, 27) et une second extrémité (26b, 27b) de la courbe supérieure (26, 27) se situe entre 55 % et 90 % de la longueur (a6) d'une ligne droite horizontale entre un axe de symétrie vertical (K) du canal de guidage (7) et un maximum (24c, 25c) d'une courbe latérale (24, 25) et/ou une ligne droite horizontale de liaison (30) entre deux points d'extrémité (26a, 26b ; 27a, 27b) d'une courbe supérieure (26, 27) dans la seconde moitié (II) est supérieure à une distance verticale (a1) entre le maximum (26c, 27c) d'une courbe supérieure (26, 27) et la ligne droite horizontale de liaison (30) d'un facteur supérieur ou égal à 2.

5. Pointe d'injecteur selon l'une quelconque des revendications précédentes,
**caractérisée en ce que**
dans la seconde moitié (II), au moins dans des sections, une distance verticale (a2) entre un minimum (23c) de la courbe inférieure (23) et un minimum (26a, 27a ; 31) du contour supérieur entre les deux courbes supérieures (26, 27) est plus importante, en particulier plus importante d'au moins un facteur de 5, qu'une distance verticale (a1) entre le maximum (26c, 27c) d'une courbe supérieure (26, 27) et une ligne droite horizontale de liaison (30) par le minimum (26a, 27a ; 31) du contour supérieur.

6. Pointe d'injecteur selon l'une quelconque des revendications précédentes,
**caractérisée en ce que**
au moins dans la seconde moitié (II), un rapport de longueur entre une ligne droite verticale de liaison (29) reliant les points d'extrémité (24a, 24b ; 25a, 25b) d'une courbe latérale (24, 25) et une distance verticale (a7) entre un maximum (26c ; 27c) d'une courbe supérieure (26, 27) et un minimum (23c) de la courbe inférieure (23) se situe entre 0,6 et 0,8, en particulier entre 0,65 et 0,78.

7. Pointe d'injecteur selon l'une quelconque des revendications précédentes,
**caractérisée en ce que**
les courbes latérales (24, 25) et les courbes supérieures (26, 27) dans la seconde moitié (II) du canal de guidage (7) sont formées de telle sorte qu'une distance (a2) entre le minimum inférieur (23c) et un creux de contour (31), en particulier un minimum (26a, 27a) dans le contour supérieur, entre les deux courbes supérieures (26, 27) soit au moins 10 %, en particulier au moins 20 %, plus importante qu'une épaisseur verticale (a8) de la lentille intraoculaire (12) à transporter dans le canal de guidage à cette position du creux de contour (31).

8. Pointe d'injecteur selon l'une quelconque des revendications précédentes,
**caractérisée en ce que**
le rayon d'une courbe supérieure (26, 27) depuis une première extrémité (26a, 27a) jusqu'à une second extrémité (26b, 27b) vu le long de l'axe longitudinal (A) de la pointe d'injecteur (4) est formé décroissant, en particulier formé décroissant de manière continue, et/ou le rayon d'une courbe latérale (24, 25) depuis une première extrémité (24a, 25a) jusqu'à une second extrémité (24b, 25b) vu le long de l'axe longitudinal (A) de la pointe d'injecteur (4) est formé croissant, en particulier formé croissant de manière continue et/ou un rayon d'une courbe supérieure (26, 27) dans la seconde moitié (II) de la pointe d'injecteur (4) vu le long de l'axe longitudinal (A) est formé entre 50 % et 150 % d'un rayon d'une courbe latérale (24, 25).

9. Pointe d'injecteur selon l'une quelconque des revendications précédentes,
**caractérisée en ce que**
le rayon d'une courbe latérale (24, 25) vu sur toute la longueur du canal de guidage (7) est supérieur ou égal à 0,6 mm, en particulier est compris entre 0,7 mm et 1 mm et/ou le rayon d'une courbe supérieure (26, 27) au moins dans la seconde moitié (II) du canal de guidage (7) est supérieur à 0,6 mm, en particulier est compris entre 0,6 mm et 0,8 mm.

10. Pointe d'injecteur selon l'une quelconque des revendications précédentes,
**caractérisée en ce que**
le canal de guidage (7) comporte une paroi façonnée (8) délimitant l'espace interne du canal (7), au moyen duquel une lentille intraoculaire (12) est pliée au moins à un endroit dans une première moitié avant (I) vue le long de l'axe longitudinal (A) du canal de guidage (7) de telle sorte que des bords latéraux opposés (17, 18) de la lentille intraoculaire (12) restent sur le côté supérieur (16) de la partie optique (13) de la lentille intraoculaire (12).

11. Dispositif injecteur ayant une pointe d'injecteur (4) selon l'une quelconque des revendications précédentes.

12. Dispositif injecteur selon la revendication 11,
**caractérisé en ce que**
il comporte un tube d'injecteur (2), sur lequel la pointe d'injecteur (4) est disposée au niveau de l'extrémité avant (9a), et dans le tube d'injecteur (2), en particulier dans une cassette (3), qui peut être fixée dans le tube d'injecteur (2) devant la point d'injecteur (4), un espace de réception (9) pour la lentille intraoculaire (12) est formé, dans lequel l'espace de réception (9) est formé effilé, en particulier effilé de manière continue, vers la pointe d'injecteur (4) dans une vue en coupe longitudinale et/ou l'espace de réception (9) comporte des parois de confinement (11a, 11b), qui sont formées de telle sorte que la lentille intraoculaire (12) soit disposée inclinée vers le fond dans l'espace de réception (9) dans sa position de repos, dans lequel une paroi de plafond (11a) de l'espace de réception (9) comporte une région de guidage (20) pour un piston plongeur (9) pour faire avancer la lentille (12) en dehors de l'espace de réception (9) dans la pointe d'injecteur (4) et le piston plongeur (19) est plié vers le bas par la région de guidage (20) lors d'un mouvement dans l'espace de réception (9).
